(19) Europäisches Patentamt
European Patent Office
Office européen des brevets

(11) Publication number: **0 232 869 B1**

(12) **EUROPEAN PATENT SPECIFICATION**

(45) Date of publication of patent specification: **12.05.93**
(51) Int. Cl.[5]: **C07D 405/14**

(21) Application number: **87101663.0**

(22) Date of filing: **06.02.87**

(54) **Method for solidifying triglycidyl isocyanurate.**

(30) Priority: **13.02.86 JP 29575/86**

(43) Date of publication of application:
**19.08.87 Bulletin 87/34**

(45) Publication of the grant of the patent:
**12.05.93 Bulletin 93/19**

(84) Designated Contracting States:
**AT CH DE FR GB IT LI NL**

(56) References cited:
**DE-A- 1 595 438**
**FR-A- 1 570 826**
**GB-A- 2 113 222**

**CHEMICAL ENGINEERING PRACTICE, vol. 6: Fluid Systems II, 1958, Edited by H.W. CREMER et al., page 405, Butterworths Scientific Publications, London, GB;**

**ENCYCLOPEDIA OF CHEMICAL TECHNOLOGY, 1980, Edited by KIRK-OTHMER, third edition, vol. 7, pages 260-261, John Wiley & Sons, New York, US;**

(73) Proprietor: **NISSAN CHEMICAL INDUSTRIES LTD.**
**3-7-1, Kanda Nishiki-cho**
**Chiyoda-ku Tokyo(JP)**

(72) Inventor: **Yasuo, Takakuwa Nissan Chem. Ind. Ltd.**
**Chuo Kenkyusho, 722-1, Tsuboi-cho**
**Funabashi-shi Chiba-ken(JP)**
Inventor: **Hisao, Ideda Nissan Chem. Ind. Ltd.**
**Chuo Kenkyusho, 722-1, Tsuboi-cho**
**Funabashi-shi Chiba-ken(JP)**

(74) Representative: **Wächtershäuser, Günter, Dr.**
**Tal 29**
**W-8000 München 2 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.6/3.3.1)

## Description

The present invention relates to a method for producing pulverizable solid triglycidyl isocyanurate from molten triglycidyl isocyanurate formed by the reaction of isocyanuric acid with epichlorohydrin.

The main component of technical grade triglycidyl isocyanurate is known to be tris (2,3-epoxypropyl)isocyanurate represented by the formula:

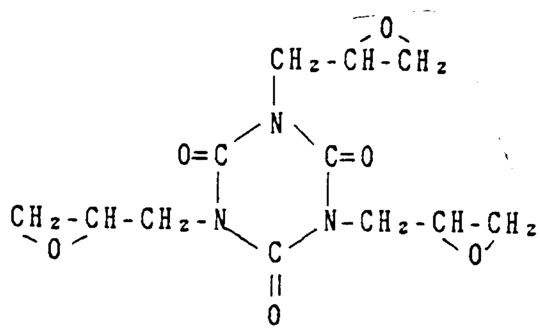

(I)

It is used primarily as a curing agent for polyester type powder paints, and in the field of sealing compounds for electronic materials and in other fields.

For the production of triglycidyl isocyanurate, a process is known wherein isocyanuric acid is reacted with an excess amount of epichlorohydrin at a high temperature in the presence of a quaternary ammonium salt, the reaction product is cooled and subjected to a dehydrochlorination reaction with a strong base such as sodium hydroxide, the by-product is removed by filtration or washing with water, and excess epichlorohydrin is distilled off under reduced pressure to obtain colorless transparent viscous triglycidyl isocyanurate.

Several methods are known to convert viscous triglycidyl isocyanurate into powder or granule which is an advantageous product form for various applications.

For example, there have been proposed a method wherein viscous triglycidyl isocyanurate is simply cooled to obtain solid substance, which is then pulverized (U.S. Patent 3,547,918), a recrystallization method of viscous triglycidyl isocyanurate (Japanese Examined Patent Publication No. 1989/1967), and a crystallization method wherein viscous triglycidyl isocyanurate is added to a solvent for precipitation (U.S. Patent 3,483,168).

In recent years, there has been a strong demand for reducing residual epichlorohydrin as far as possible i.e. to a level of not higher than 1,000 ppm, when triglycidyl isocyanurate is to be used as a curing agent for polyester type powder paints.

As a method for removing residual epichlorohydrin, it is common to employ a method wherein triglycidyl isocyanurate formed by the reaction of isocyanuric acid and epichlorohydrin, is treated at a high temperature under a highly vacuumed condition to remove epichlorohydrin. U.S. Patent 3,547,918 discloses that when such triglycidyl isocyanurate is dried at 90-100°C under a highly vacuumed condition for 20-30 minutes, the dried product will completely solidify in about one hour. However, the removal of epichlorohydrin under such a condition is not still adequate. In order to efficiently remove eichlorohydrin adequately, i.e. to a level of the residual epichlorohydrin being not higher than 1,000 ppm, preferably not higher than 100 ppm, it is usually required to heat the formed triglycidyl isocyanurate at a temperature higher than its melting point i.e. at least 92°C. For example, U.S. Patent 4,395,542 discloses that such triglycidyl isocyanurate was treated at 122-167°C under 8 millibar to reduce the residual epichlorohydrin to a level of 4-459 ppm. However, if treated at such a high temperature, the completely molten highly viscous triglycidyl isocyanurate will hardly be solidified, or the solidification speed of the viscous triglycidyl isocyanurate will be very slow, and it takes at least a few days to obtain a pulverizable solid product by the method wherein the viscous triglycidyl isocyanurate is simply cooled.

It is believed that such triglycidyl isocyanurate is hardly solidified because it contains from 5 to 15% by weight of other substances such as diglycidyl isocyanurate, products formed by the ring opening of epoxy rings, etc.

On the other hand, the above-mentioned recrystallization and precipitation methods involve cumbersome operations. Besides, they are not economically advantageous, since the yield of the triglycidyl isocyanurate product is low.

The present inventors have conducted extensive researches to develop a method for directly and efficiently solidifying molten viscous triglycidyl isocyanurate formed by the reaction of isocyanuric acid with epichlorohydrin, and have finally accomplished the present invention.

The present invention provides a method for producing pulverizable solid triglycidyl isocyanurate as defined in claim 1 which comprises dispersing triglycidyl isocyanurate powder as seed to molten triglycidyl isocyanurate formed by the reaction of isocyanuric acid with epichlorohydrin, at a temperature of from 70 to 90 °C and cooling the dispersion.

Now, the present invention will be described in detail with reference to the preferred embodiments.

The triglycidyl isocyanurate to be solidified by the present invention is viscous molten triglycidyl isocyanurate obtained by the reaction of isocyanuric acid with epichlorohydrin.

More specifically, it is molten triglycidyl isocyanurate obtained by removing, by filtration or washing with water, by-products such as sodium chloride from the reaction product obtained by the reaction of isocyanuric acid and epichlorohydrin, and further removing epichlorohydrin.

The molten triglycidyl isocyanurate preferably has an epoxy value of at least 8.8 and an epichlorohydrin content of not higher than 1,000 ppm, preferably not higher than 100 ppm, more preferably not higher than 50 ppm.

The triglycidyl isocyanurate used as seed in the present invention includes various types of triglycidyl isocyanurate.

In general, triglycidyl isocyanurate is known to be an α-type (melting point: 103-105), a β-type (melting point: 156-158 °C) or a mixture thereof (mixture of α- and β-types). These different types of triglycidyl isocyanurate may be used individually or in combination as a mixture.

Technical grade of triglycidyl isocyanurate formed by the reaction of isocyanuric acid with epichlorohydrin is usually a mixture of α- and β-types including impurities such as diglycidyl isocyanurate, products formed by the ring opening of epoxy rings and usually has a melting point of from 92 to 115 °C.

Triglycidyl isocyanurate obtained by the method of the present invention as well as commercially available triglycidyl isocyanurate and triglycidyl isocyanurate (mixture of α- and β-types) recrystallized from methanol, may also be used as seed.

The smaller the particle size of the triglycidyl isocyanurate powder as seed is, the greater the effect as seeds for crystal growth is, and the faster the solidification becomes.

The particle size is usually at most 4 mesh pass (4.8mm or less), preferably at most 12 mesh pass (1.4mm or less), more preferably at most 20 mesh pass (0.8mm or less). The seed is dispersed in an amount of from 0.5 to 40% by weight, preferably from 5 to 40% by weight, more preferably from 10 to 30% by weight, relative to the total amount of the triglycidyl isocyanurate powder as seed and the molten triglycidyl isocyanurate. If the amount is less than 0.5% by weight, the solidification time tends to be too long. On the other hand, if the amount exceeds 40% by weight, the mixture tends to be a highly viscous slurry, whereby the mixing operation tends to be difficult, such being disadvantageous from the practical point of view.

The temperature for dispersing seed to the molten triglycidyl isocyanurate is usually from 70 to 115 °C, preferably from 75 to 90 °C, more preferably from 80 to 88 °C. When the dispersing temperature is within a range of from 92 to 115 °C, the seed may gradually melt and the effect of the seed may accordingly be reduced. Nevertheless, the operation can be conducted within such a temperature range by adding seed afresh. If the dispersing temperature is lower than 70 °C, the dispersion of the seed tends to be difficult, such being disadvantageous from the practical point of view.

The shorter the time for the dispersion of seed, the better so long as uniform dispersion can be obtained. If the time for the dispersing and mixing is prolonged, it is likely that seed dissolves and the viscosity of the system increases. Usually, the dispersing and mixing operation is conducted within 1 hour.

The dispersion thus obtained is then cooled to 60 °C over a period of at least 2 minutes, preferably from 3 to 30 minutes.

Triglycidyl isocyanurate solidified by the method of the present invention can readily be pulverized by a conventional pulverizer to any desired particle size.

Now, the present invention will be described in further detail with reference to Examples and Comparative Examples.

PREPARATION EXAMPLE:

Preparation of triglycidyl isocyanurate

Viscous molten triglycidyl isocyanurate used in the present invention was prepared by the following method.

1.29 kg of isocyanuric acid, 13.88 kg of epichlorohydrin and 0.05 kg of tetramethylammonium chloride, were stirred at a temperature of from 100 to 120 °C for 4 hours.

Then, the mixture was cooled to 50 °C, and 2.64 kg of a 50% sodium hydroxide aqueous solution was added over a period of 4 hours while azeotropically removing water under a reduced pressure of from 80 to 100 mmHg at a temperature of from 45 to 50 °C. Sodium chloride formed as a by-product and impurities were removed by washing with water, and excess epichlorohydrin was distilled off under reduced pressure. Further, nitrogen purging was conducted at 110 °C in a molten state under a reduced pressure of 10 mmHg for 1 hour to reduce the epichlorohydrin content to a level of not higher than 80 ppm. Thus, 2.55 kg of colorless transparent viscous molten triglycidyl isocyanurate (α-type 69%, β-type 22%, others 9% by weight) was obtained.

The epoxy value was 9.8, the chlorine content was 1.2% by weight, and the yield based on the isocyanuric acid was 86%.

COMPARATIVE EXAMPLE 1 (according to US-A-3,483,168)

250 g of methanol was added to 100 g of the viscous molten triglycidyl isocyanurate obtained in the Preparation Example, and the mixture was stirred at 60 °C for 30 minutes. Then, the mixture was subjected to precipitation at 0 °C, followed by filtration to obtain 86 g of a white powder of triglycidyl isocyanurate (α-type 74%, β-type 25%, others 1% by weight). The epoxy value was 9.9, the chlorine content was 0.2% by weight, and the yield based on the isocyanuric acid was 76%.

COMPARATIVE EXAMPLE 2

100 g of the viscous molten triglycidyl isocyanurate obtained in the Preparation Example was transferred onto a metal sheet in the same manner as in Example 4 of U.S. Patent 3,547,918 and left to cool at room temperature for 1 day and night, whereby it did not solidify but became a very viscous paste-like substance. Further, it was left to stand at room temperature for 1 week, but it did not solidify to such an extent that it was pulverizable.

EXAMPLE 1

Into a 500 ml reaction flask equipped with a stirrer and a thermometer, 320 g of the viscous molten triglycidyl isocyanurate obtained in the Preparation Example, was charged. Then, 50 g of triglycidyl isocyanurate powder, (12 mesh pass (1.4mm or less), melting point: 92-115 °C (α-type 69%, β-type 22%, others 9% by weight)) was added as seed. The mixture was uniformly dispersed at a temperature of from 82 to 84 °C under stirring for 10 minutes. Then, the dispersion was transferred to a flat bat and left to cool at room temperature. In 2 minutes, the temperature of the dispersion dropped to 60 °C and solidified in 8 minutes to such an extent that it was pulverizable. The Durometer hardness (A-model) was from 80 to 90.

EXAMPLE 2

The same treatment as in Example 1 was conducted except that 50 g of triglycidyl isocyanurate powder (12 mesh pass (1.4mm or less), melting point: 92-115 °C) was added as seed to 320 g of the viscous molten triglycidyl isocyanurate obtained in the Preparation Example, and uniformly dispersed at 95 °C under stirring for 10 minutes. Then, the dispersion was transferred to a flat bat and left to cool at room temperature for 24 hours to obtain a solidified product. The Durometer hardness (A-model) of the solidified product was from 80 to 90.

EXAMPLES 3 to 8

By using 320 g of the viscous molten triglycidyl isocyanurate obtained in the Preparation Example, the solidification was conducted in the same manner as in Example 1 under the conditions as identified in Table 1. In Example 8, the particle size of the seed was 42 mesh pass (360μm or less). In other Examples, the particle size of the seed was 12 mesh pass (1.4mm or less).

Table 1

| Example | Seed | Mixing temp. | Dispersing time | Solidification time |
|---|---|---|---|---|
| 1 | 50 g | 82-84 (°C) | 10 min. | 10 min. |
| 2 | 50 " | 95 " | 10 " | 24 hrs. |
| 3 | 30 " | 82-84 " | 10 " | 25 min. |
| 4 | 60 " | 82-84 " | 10 " | 5 " |
| 5 | 50 " | 82-84 " | 60 " | 20 " |
| 6 | 50 " | 90 " | 10 " | 30 " |
| 7 | 15 " | 82-84 " | 10 " | 40 " |
| 8 | 15 "* | 82-84 " | 10 " | 5 " |

* Particle size of the seed is 42 mesh pass (360μm or less)

It is evident from Table 1 that as compared with the conventional methods represented by the Comparative Examples, the method of the present invention is superior in that the solidification time can be remarkably shortened.

## Claims

1. A method for producing pulverizable solid triglycidyl isocyanurate, which comprises dispersing triglycidyl isocyanurate powder having a particle size of at most 4 mesh pass (4.8 mm or less) as seed at a temperature of from 70 to 90°C into molten triglycidyl isocyanurate having an epoxy value of at least 8.8 and an epichlorohydrin content of not higher than 1000 ppm formed by the reaction of isocyanuric acid with epichlorohydrin, and cooling the dispersion to 60°C or less over a period of at least 2 minutes.

2. The method according to Claim 1, wherein the triglycidyl isocyanurate powder is dispersed in an amount of from 0.5 to 40% by weight relative to the total amount of the triglycidyl isocyanurate powder and the molten triglycidyl isocyanurate.

## Patentansprüche

1. Ein Verfahren zum Herstellen von pulverisierbarem, festem Triglycidylisocyanurat, umfassend das Dispergieren von Triglycidylisocyanurat-Pulver mit einer Teilchengröße von 4,8 mm oder weniger (entsprechend höchstens Durchgang durch 4 Maschen) als Kristallkeime bei einer Temperatur von 70

bis 90°C in geschmolzenem Triglycidylisocyanurat mit einem Epoxywert von mindestens 8,8 und einem Epichlorhydringehalt von nicht mehr als 1000 ppm, das durch Umsetzung von Isocyanursäure mit Epchlorhydrin gebildet wurde, und Abkühlen der Dispersion auf 60°C oder weniger während einer Zeitdauer von mindestens 2 Minuten.

**2.** Das Verfahren nach Anspruch 1, worin das Triglycidylisocyanurat-Pulver in einer Menge von 0,5 bis 40 Gew.-%, bezogen auf die Gesamtmenge des Triglycidylisocyanurat-Pulvers und des geschmolzenen Triglycidylisocyanurats, dispergiert wird.

## Revendications

**1.** Procédé de fabrication d'un isocyanurate de triglycidyle solide, pulvérisable, qui comprend la dispersion d'une poudre d'isocyanurate de triglycidyle ayant une dimension de particule lui permettant de passer à travers un tamis d'au plus 4 mesh (4,8 mm ou moins) en tant que germe, à une température de 70 à 90°C dans de l'isocyanurate de triglycidyle fondu ayant un indice d'époxyde d'au moins 8,8 et une teneur en épichlorhydrine non supérieure à 1000 ppm, formé par la réaction d'acide isocyanurique avec de l'épichlorhydrine, et le refroidissement de la dispersion à 60°C, ou au-dessous, sur une période de temps d'au moins 2 minutes.

**2.** Procédé selon la revendication 1, dans lequel la poudre d'isocyanurate de triglycidyle est dispersée dans une quantité de 0,5 à 40% en poids par rapport au poids total de la poudre d'isocyanurate de triglycidyle et de l'isocyanurate de triglycidyle fondu.